# EUROPÄISCHE PATENTSCHRIFT

(11) **EP 0 632 995 B1**
(45) Veröffentlichungstag und Bekanntmachung des Hinweises auf die Patenterteilung: **21.04.1999**
(21) Anmeldenummer: 94109424.5
(22) Anmeldetag: 17.06.1994
(51) Int. Cl.: A61B 6/14, A61B 6/00

(54) **Zahnärztliche Röntgendiagnostikeinrichtung**
Dental X-ray diagnostic device
Appareil de radiodiagnostic dentaire

(30) Priorität: 06.07.1993 DE 4322483; 30.05.1994 EP 94108334
(43) Veröffentlichungstag der Anmeldung: 11.01.1995
(73) Patentinhaber: Sirona Dental Systems GmbH & Co.KG, 64625 Bensheim (DE)
(72) Erfinder: Franetzki, Manfred, Dr.-Ing. Dipl.-Phys., D-64625 Bensheim (DE); Günther, Werner, Ing. grad., D-64625 Bensheim (DE); Plötz, Josef, Dr. rer. nat. Dipl.-Phys., D-64625 Bensheim (DE)
(74) Vertreter: Isenbruck, Günter, Dr.

(56) Entgegenhaltungen:
- EP-A- 0 035 307
- EP-A- 0 166 567
- EP-A- 0 229 971
- EP-A- 0 262 500
- EP-A- 0 279 293
- DE-A- 3 930 022
- US-A- 5 214 686

## Beschreibung

Die Erfindung bezieht sich auf eine zahnärztliche Röntgendiagnostikeinrichtung zur Erstellung von tomosynthetischen Bildern von Objekten eines Patienten.

Bei der Tomosynthese wird ein Objekt aus verschiedenen Projektionsrichtungen durchstrahlt und danach die entstehenden 2D-Bilder in einem Rechner zu Schichtaufnahmen und 3D-Bildern verarbeitet. Die Aufnahme der Bilder kann in herkömmlicher Weise auf Filmmaterial erfolgen oder auch über elektronische Bildwandler (Röntgenbildverstärker, CCD-Kamera oder Digitalkamera auf Basis von amorphem Silizium).

Der im Anspruch 1 angegebenen Erfindung liegt die Aufgabe zugrunde, eine zahnärztliche Röntgendiagnostikeinrichtung anzugeben, mit der es möglich ist, mit relativ geringem Aufwand tomosynthetische Aufnahmen erstellen zu können. Die Erfindung fußt auf der Erkenntnis, die wesentlichen Komponenten der aus der Orthopanthomographie und der Cephalometrie bekannten Geräte zu nutzen und durch geeignete Zusatzelemente so zu modifizieren, daß die vorgenannten Aufnahmen mit geringem Aufwand zu erstellen sind. Eine weitere Zielsetzung liegt in der Abwandlung eines Panorama-Röntgengerätes zum Zwecke der Erzeugung von Fernröntgenbildern des Schädels herkömmlicher Art, d.h. Darstellung der Summenabsorption bei kompletter Durchstrahlung des Schädels unter Verwendung tomosynthetischer Rechenverfahren.

Hinsichtlich des Aufbaus herkömmlicher Panorama(PAN)-Röntgenaufnahmevorrichtungen sowie Vorrichtungen zur Erstellung von Schädelaufnahmen (Ceph-Aufnahmen) wird auf die EP-A-0 229 308 verwiesen.

Des weiteren wird auf DE-3 930 022 A1 und US-PS 5 214 686 verwiesen. In dem erstgenannten Dokument wird eine Panorama-Röntgenaufnahmevorrichtung beschrieben, die auch Aufnahmen vom Schädel eines Patienten erlaubt. Hierzu ist in dem Röntgenstrahlengenerator eine Blendenanordnung mit zwei parallelen rechteckigen Maskenplatten untergebracht, die mit verschiedenen, in Längsrichtung der Platten aufeinanderfolgenden Strahlendurchgangsöffnungen ausgestattet sind. Je nachdem, ob Panorama-Röntgenaufnahmen oder Schädelaufnahmen erstellt werden sollen, werden die Maskenplatten seitlich verschoben und auf die entsprechenden Blendenöffnungen eingestellt.

In der US-Patentschrift wird ein Verfahren und eine Vorrichtung zur dreidimensionalen Darstellung von Panorama-Aufnahmen vom Schädel eines Patienten beschrieben. Röntgenstrahlenquelle und Detektoreinheit sind hier anders ausgebildet als bei den vorbeschriebenen Vorrichtungen. Der zunächst breitfächerig erzeugte Röntgenstrahl wird mittels eines Kollimators in eine Vielzahl von vertikalen, sehr schmalen, eng begrenzten Strahlenbündeln unterteilt. Diesen eng begrenzten Strahlenbündeln sind auf der Detektorseite entsprechend viele, ebenfalls vertikal angeordnete Detektor-Arrays zugeordnet. Strahlenquelle und Detektoreinheit werden in konventioneller Weise, wie bei Panoramaaufnahmen üblich, um den Patientenkopf herum bewegt. Aus den vielen dabei erzeugten, unter jeweils einem anderen Winkel aufgenommenen zweidimensionalen Bildern wird rechnerisch ein dreidimensionales Projektionsbild geschaffen.

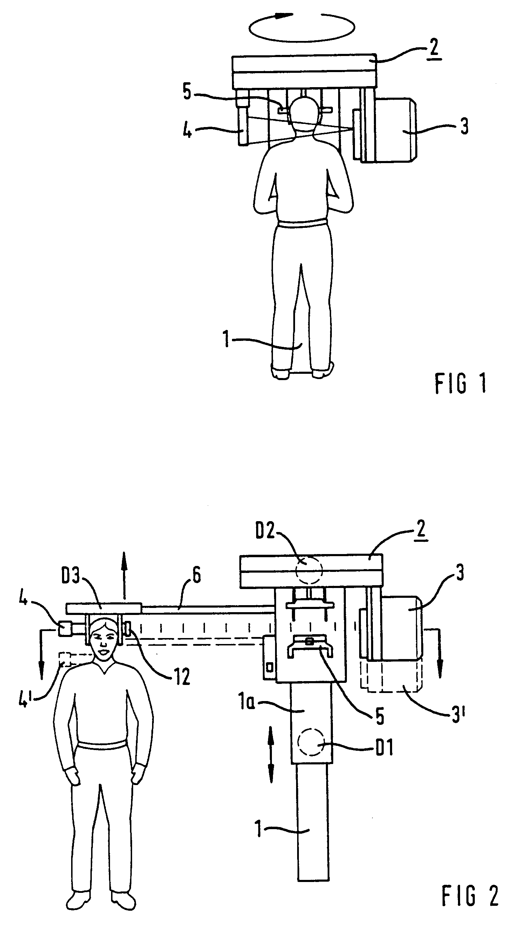

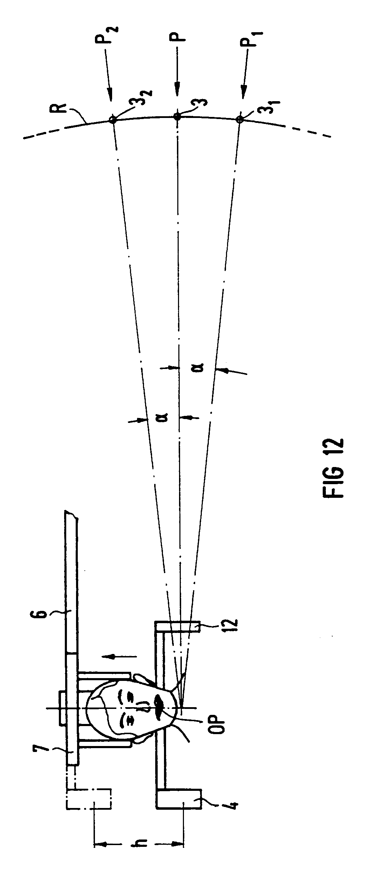

Anhand der Figuren 1 bis 11 der Zeichnung wird zunächst der Grundaufbau verschiedener Varianten beschrieben, mit denen sich Röntgenaufnahmen ohne Film und Filmkassette erstellen lassen. Die darauffolgenden Figuren 12 bis 17 zeigen Modifikationen dieser Ausführungsformen, mit denen sich mit relativ geringem Aufwand die eingangs genannten tomosynthetischen Röntgenaufnahmen erstellen lassen.

Die Figur 1 zeigt in einer Prinzipdarstellung ein zahnärztliches Röntgendiagnostikgerät zur Erstellung von Panorama-Schichtaufnahmen, nachfolgend kurz mit PAN-Aufnahmen bezeichnet. Das Gerät enthält eine in der Höhe verstellbare Tragsäule 1, an der eine Dreheinheit 2 gehaltert ist, die Träger einerseits einer Röntgenstrahlenquelle 3 und andererseits diametral dazu einer Röntgenzeilenkamera 4 ist. Mit 5 ist eine (erste) Kopfhalte- und Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte- und Positioniereinrichtung sind bekannt und beispielsweise in der EP-0 229 308 beschrieben.

Die Figur 2 zeigt das gleiche Grundgerät, bestehend aus höhenverstellbarer Tragsäule 1, Dreheinheit 2 und Röntgenstrahler 3, ergänzt jedoch durch eine am Gerät adaptierbare Vorrichtung, mit der sich Schädelfernaufnahmen, nachfolgend kurz Ceph-Aufnahmen, erstellen lassen. Bevor die genannte Vorrichtung näher erläutert wird, sei noch erwähnt, daß die Tragsäule 1 mittels eines Antriebes D1 in der angegebenen Pfeilrichtung höhenverstellbar ausgebildet ist, daß weiterhin in bekannter Weise die Dreheinheit 2 mittels eines oder mehrerer Antriebe D2 gedreht und geschwenkt werden kann, um eine PAN-Aufnahme machen zu können. Einzelheiten hierzu ergeben sich aus der bereits genannten EP-0 229 308.

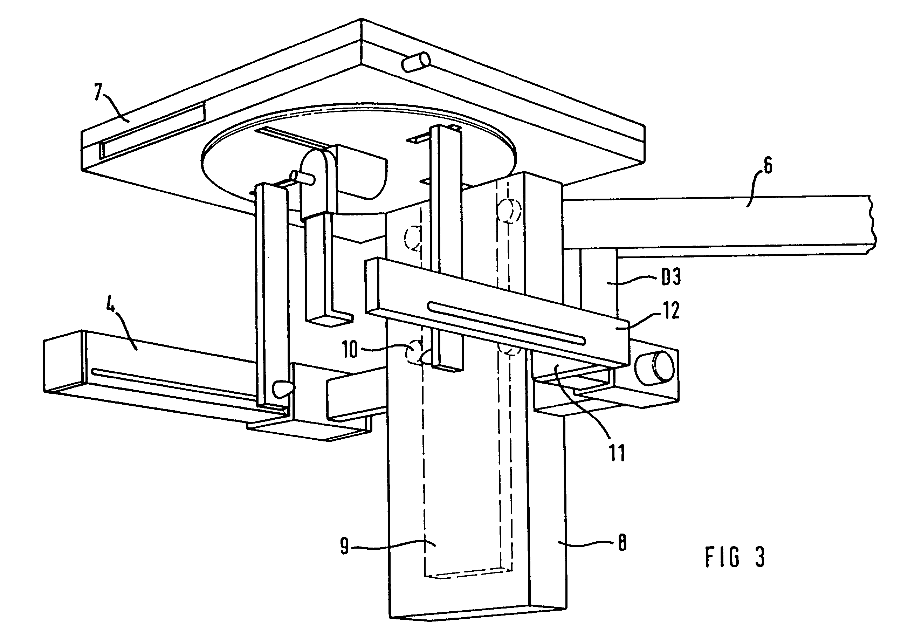

Die Figur 3 zeigt die vorgenannte Vorrichtung zur Erstellung von Ceph-Aufnahmen in einer schaubildlichen Darstellung.

Am höhenverstellbaren Teil 1a der Tragsäule 1 (Fig. 2) ist ein Auslegerarm 6 befestigt, der eine (zweite) Kopfhalte- und Positoniereinrichtung 7 trägt. Der Auslegerarm 6 umfaßt ein Gehäuse 8, an dem ein Schwert 9, welches die Kopf-halte- und Positioniereinrichtung trägt, mit Hilfe von im Gehäuse 8 angeordneten Führungsrollen 10 verstellbar gelagert ist. Die Zeilenkamera 4 ist mittels eines Querträgers 11 mit einer Vorblende 12 verbunden, die dazu dient, den an sich schon in bekannter Weise von der der Röntgenstrahlenquelle 3 benachbarten Sekundärblende begrenzten Fächerstrahl nochmals exakt auf die Schlitzbreite und -länge der nachfolgend noch näher erläuterten Zeilenkamera zu justieren.

Im Gegensatz zum Ausführungsbeispiel nach Figur 1 ist die Zeilenkamera bei der Ausführung nach Figur 2 nicht vertikal, sondern waagerecht angeordnet. Eine entsprechend ausgebildete Halterung ist in Figur 5 dargestellt.

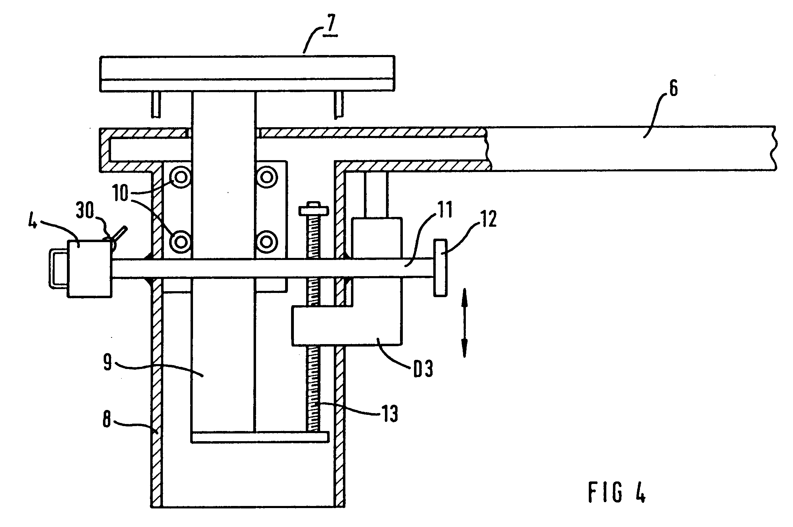

Wie aus Figur 4, die die Vorrichtung in Frontansicht und teilweise im Schnitt zeigt, ersichtlich, befindet sich am Schwert 9, welches die Kopfhalte- und Positioniereinrichtung trägt, eine Gewindespindel 13, die mit einem allgemein mit D3 bezeichneten Getriebemotor zusammenwirkt. Der Getriebemotor D3 ist entweder am Gehäuse 8 oder am Tragarm 6 befestigt. Wie später noch näher erläutert, wird mit Hilfe der aufgezeigten Verstellanordnung mit dem Antrieb D3 erreicht, daß, wenn die Röntgenstrahlenquelle 3 zusammen mit der Zeilenkamera 4 in der Vertikalen bewegt wird, die Kopfhalte- und Positioniereinrichtung 7 während der Ceph-Aufnahme effektiv keine Bewegung ausführt, d.h. ortsfest im Raum gehalten wird.

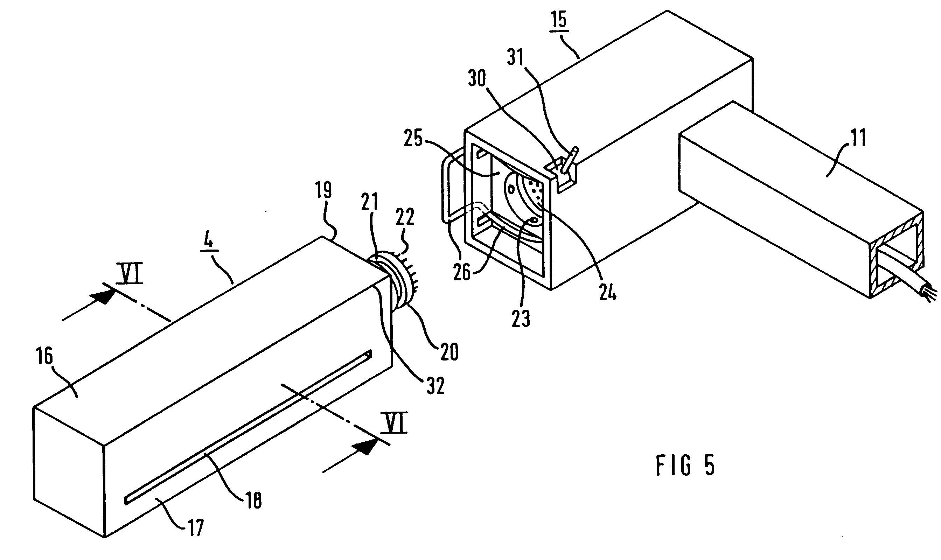

Die Figur 5 zeigt in einer schaubildlichen Explosionsdarstellung einerseits die Zeilenkamera 4 und andererseits eine mit 15 bezeichnete Halterung, die im vorliegenden Anwendungsfalle für die Ausführung nach Figur 2 am Querträger 11 befestigt ist. Im Falle der Version nach Figur 1 (für PAN-Aufnahmen) ist eine gleich ausgebildete Halterung (ohne Querträger 11) senkrecht an der Dreheinheit 2 (Figur 1) befestigt.

Die Zeilenkamera 4 enthält ein längliches Gehäuse 16, welches im Ausführungsbeispiel aus einem Vierkantrohr besteht und in der vorderen, der Strahlenquelle 3 zugewandten Seitenfläche 17 einen Schlitz 18 aufweist. Der Schlitz 18 befindet sich im unteren Drittel der Seitenfläche 17, wodurch die Zeilenkamera in eine vergleichsweise tiefe Ausgangsposition (sh. gestrichelte Darstellung in Fig. 2) gefahren werden kann.

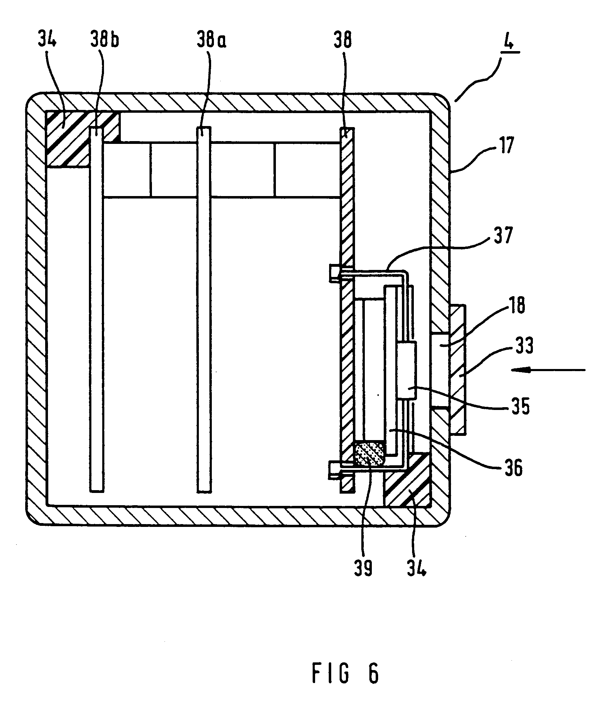

Wie aus Figur 6 noch näher hervorgeht, befindet sich hinter dem Schlitz 18 im Innern des Profilrohres 16 ein strahlenempfindlicher Detektor in Form eines zweidimensionalen CCD-Sensors. An der einen Stirnseite 19 befindet sich ein zapfenförmiges Anschlußelement 20, welches mechanische und elektrische Anschlußmittel für eine elektrische und mechanische Verbindung mit dem Halter 15 aufweist. Die mechanischen Anschlußmittel enthalten eine Ringnut 21, die mit einer Kugelrastung 23 zusammenwirkt. Die elektrischen Anschlußmittel bestehen aus einem Mehrstiftstecker 22, der mit einer Steckbuchse 24 im Halter 15 zusammenwirkt. Die Steckstifte 22 sind mit dem bereits erwähnten Zeilendetektor und einer weiteren, im Inneren der Zeilenkamera 4 befindlichen Elektronik verbunden. Der Halter 15 ist so aufgebaut, daß bei aufgesetztem Zeilendetektor die Stirnseite 19 der Zeilenkamera 4 einer stirnseitigen Anschlußfläche 25 der Halterung 15 gegenübersteht.

Damit das Lösen der Zeilenkamera 4 von der Halterung 15 erleichtert ist, insbesondere ein Verkanten und damit die Gefahr einer Beschädigung der hochempfindlichen elektrischen Kontakte vermieden wird, ist eine Auswurfeinrichtung 26 vorgesehen. Diese besteht in der vorliegenden Ausführungsform aus einem Bügel, der in einem Schlitz der Gehäusewandung des Halters 15 nach außen geführt ist. Wird der Bügel bei aufgesetzter Zeilenkamera betätigt, drücken anliegende Bügelteile gegen die Stirnfläche 19 und üben so eine zentrische Kraft auf die Fläche aus, wodurch die Verbindung leicht gelöst werden kann.

Mit 30 ist eine Zentriereinrichtung bezeichnet, die einen exzentrisch im Gehäuse der Halterung gelagerten Hebel 31 enthält. Nach Einsetzen der Zeilenkamera 4 in den Halter 15 wird der Exzenterhebel 31 betätigt, wodurch eine Fläche des Exzenters auf die in der Figur mit 32 bezeichnete Kante des Gehäuses drückt und dieses in definierter, reproduzierbarer Position hält. Obgleich im vorliegenden Ausführungsbeispiel das Gehäuses einteilig ausgebildet ist, kann das Gehäuse auch mehrteilig ausgeführt sein, wobei der eine, den Detektor tragende Gehäuseteil dann in der vorgenannten Weise zentriert wird. Damit kann der Detektor unabhängig vom Kameragehäuse und dessen etwaigen Montage- und Fertigungstoleranzen in bezug auf den Halter fixiert werden.

Aus Figur 6, die einen Schnitt entlang der Linie VI-VI in Figur 5 zeigt, geht der prinzipielle Aufbau der Zeilenkamera hervor. Das Gehäuse ist lichtdicht ausgebildet; der Schlitz 18 ist stirnseitig von einer lichtdichten, aber röntgenstrahlendurchlässigen Kunststoffplatte 33 bedeckt. Dahinter befindet sich im Innern ein mit einer vorgeschalteten Szintillationsschicht und gegebenenfalls mit einer zwischengeschalteten Faseroptik versehener CCD-Sensor 35. Der CCD-Sensor 35 kann ein- oder mehrteilig und vorteilhafterweise als Sensormatrix aus amorphem Silizium ausgebildet sein. Ein metallischer Halter 37 verbindet den Träger 36 und das CCD-Element 35 mit einer Platine 38. Flexible Kontaktstreifen 39, z.B. aus mit Goldfasern versehenem Silicon, bewirken den elektrischen Kontakt zwischen Sensor 35 und Platine 38. Die Platine 38 enthält sämtliche, zur Ansteuerung des CCD-Sensors unmittelbar erforderlichen Bauelemente. Gegebenenfalls sind im Gehäuse noch weitere Platinen 38a, 38b angeordnet. Die von der (den) Platine(n) 38 (38a, 38b) abgehenden Leitungen führen zu den bereits erwähnten Steckstiften 22 (Fig. 5). Mit 34 sind stoßabsorbierende Elemente bezeichnet, die den Detektor 35 und die Steuerplatinen 38, 38a, 38b im Gehäuse 'schwimmend' lagern. Damit lassen sich die hochempfindlichen und teueren Teile bei einem unbeabsichtigten Herabfallen der Kamera vor Bruch bzw. Lösen der Kontaktverbindungen weitgehend schützen.

Wie eingangs bereits erwähnt, ist für PAN-Aufnahmen (Fig. 1) und Ceph-Aufnahmen (Fig. 2) das gleiche Grundgerät und ein und dieselbe Kamera verwendbar. Um die für eine Ceph-Aufnahme nötige Bildgröße zu erreichen, hat die Zeilenkamera vorteilhafterweise einen entsprechend längeren Sensor. Die Zeilenkamera kann so je nach Bedarf entweder am Ceph- oder am PAN-Halter angebracht werden. Zur Halterung der Zeilenkamera am Halter 15 sind verschiedene Möglichkeiten denkbar. Anstelle der gezeigten Kugelrastung kann auch eine Bajonettverbindung vorgesehen sein. Desgleichen kann anstelle eines Vierkantprofiles eine andere äußere Formgestaltung für das Gehäuse der Zeilenkamera vorgesehen sein.

Zum Aufnahmeprinzip wird folgendes angemerkt:

Eine PAN-Schichtaufnahme wird in der Weise erzielt, daß die beim Überstreichen des aufzunehmenden Objekts (Kiefer) gewonnenen Signale in dem zweidimensional auflösenden Detektor aufaddiert werden, wobei das Aufaddieren der Signale - falls ein CCD-Sensor verwendet wird - bereits auf dem Sensor durchgeführt werden kann, indem dieser im TDI-Modus betrieben wird. Durch diese besondere Betriebsart wird die Funktion eines bewegten Filmes nachgebildet, indem die durch Belichtung erzeugten Ladungspakete im CCD-Element entsprechend weitergetaktet werden, während ständig neue Ladungen hinzukommen. Die Taktimpulse für den TDI-Betrieb werden aus den sonst für den Filmkassettenantrieb erforderlichen Schrittmotorimpulsen abgeleitet.

Alternativ ist auch ein Aufaddieren in einer späteren Signalverarbeitungsstufe möglich.

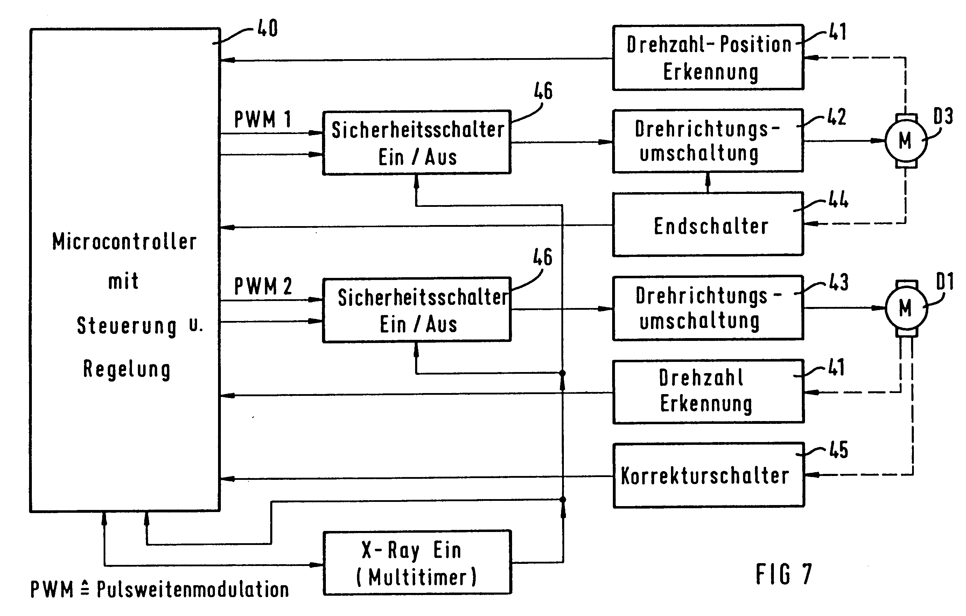

Die Ceph-Aufnahme läuft ebenfalls in Slot-Technik ab. Der Kopf eines stehenden (oder sitzenden) Patienten wird je nach Anordnung der Zeilenkamera von oben nach unten (bei waagerechter Anordnung) oder von links nach rechts (bei senkrechter Anordnung) vice versa mit einem Strahlfächer überstrichen. Dieser Strahlfächer trifft, justiert durch die bereits genannte Vorblende 11, genau auf den waagerecht angeordneten Schlitz des CCD-Sensors. Mit Hilfe des Antriebs D1 verfährt man nun das gesamte Gerät, also Röntgenstrahlenquelle 3 mit Primär- und Sekundärblende sowie Zeilenkamera 4 mit Sensor, gemeinsam von einer Ausgangsposition aus in der Vertikalen (sh. Pfeile in Fig. 2). Gleichzeitig wird die Kopfhalte- und Positioniereinrichtung 7 mit Hilfe des Antriebs D3 in Gegenrichtung gefahren, wobei die beiden Bewegungen so aufeinander abgestimmt sind, daß der Patientenkopf räumlich fixiert, d.h. ortsfest, bleibt. Die Steuerung der beiden Antriebsmotoren D1 und D3 erfolgt entsprechend dem Blockschaltbild nach Figur 7 über einen Mikroprozessor 40. Den beiden Antrieben sind Drehzahlerkennungssensoren 41, Drehrichtungsumschalter 42 sowie End- bzw. Korrekturschalter 44, 45 zugeordnet. Die über Pulsweitenmodulation erfolgte Steuerung enthält weiterhin Sicherheitsschalter 46. Eine Auswerteelektronik des Mikro-Controllers 40 erkennt, an welcher Halterung (PAN- oder Ceph-Gerät) die Kamera befestigt ist. Wird eine Ceph-Aufnahme angewählt, fährt der Antriebsmotor D3 in die Ausgangsposition, beispielsweise in die untere Verstellposition (gestrichelte Position in Fig. 2). In dieser Position spricht der Endschalter 44 an. Mittels der Höhenverstellung der Tragsäule 1 kann nun die Kopfhaltepositoniereinrichtung auf die Patientengröße eingestellt werden. Während der Ceph-Aufnahme verfährt der Antriebsmotor D3 die Kopfpositioniereinrichtung 7 nach oben, während gleichzeitig der Antriebsmotor D1 für die Tragsäule nach unten fährt. Die beiden Antriebe werden dabei so geregelt, daß die Differenz der Verstellgeschwindigkeiten gleich Null ist. Dadurch ist sichergestellt, daß der Abstand der Ohroliven und damit der Kopfpositionierung zum Fußboden konstant bleibt. Die Aufnahme ist beendet, wenn der Endschalter 44 oder ein Systemtakt-Zähler (TDI-Takt-Zähler) die obere Endlage erkennt.

Der TDI-Takt für den CCD-Sensor wird z.B. vom Antriebsmotor D1, der für die Höhenverstellung der Tragsäule vorgesehen ist, abgeleitet. Alternativ kann er auch aus den Signalen eines Positionszählers, der die Verstellung der Tragsäule direkt mißt, gewonnen werden. Der TDI-Modus dient hier nicht, wie bei einer PAN-Aufnahme, um eine Verwischung und damit eine Schichtaufnahme zu erzeugen, sondern dazu, die volle Breite des Sensors zur Bildentstehung auszunutzen. Hier entspricht also der TDI-Betrieb einem Film, der relativ zum Schlitz bewegt wird und relativ zum Patienten feststeht.

Die nachfolgenden Figuren zeigen vorteilhafte Varianten zu der in Figur 2 dargestellten Ausführungsform anhand einer Ansicht von oben (Draufsicht). Im Gegensatz zu der Ausführung nach Figur 2, bei der die Zeilenkamera 4 horizontal angeordnet ist, ist bei den Varianten nach Figuen 8 und 9 die Zeilenkamera 4 vertikal angeordnet; demnach wird auch von der Primärblende und der Vorblende ein vertikales gefächertes Strahlenbündel auf den CCD-Sensor gegeben.

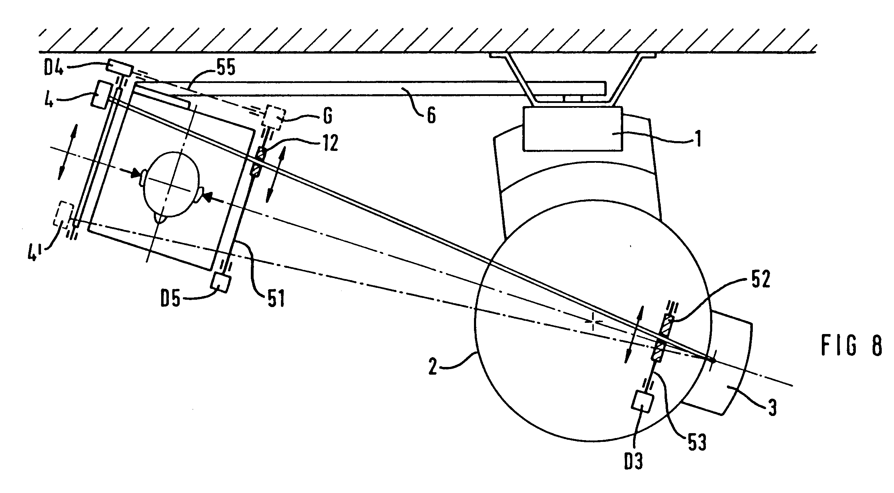

Bei der Ausführung nach Figur 8 ist sowohl die Zeilenkamera 4 als auch die Vorblende 12 in Längsführungen 50, 51 geführt, die motorisch z.B. mittels eines gemeinsamen Antriebes D4 oder, unter Zwischenschaltung eines Untersetzungsgetriebes (G) durch getrennte Antriebe D4 und D5 bewegt werden. Die Primärblende 52 ist ebenfalls mittels einer Längsführung 53 in Pfeilrichtung bewegbar. Zur Verstellung ist hier ein Antrieb D6 vorgesehen. Die Längsführungen 50, 51 und 53 können in bekannter Weise als motorisch angetriebene Gewindespindelantriebe ausgebildet sein. Die Winkelgeschwindigkeiten für die Antriebe D4, D5 und D6 sind dann, wenn jeweils getrennte Antriebe vorgesehen sind, gleich. Wenn für Längsführungen 50 und 51 ein gemeinsamer Antrieb vorgesehen werden soll, müssen die beiden Längsführungen über eine weitere Spindel 55 unter Zwischenschaltung eines geeigneten Untersetzungsgetriebes (G) miteinander gekoppelt sein.

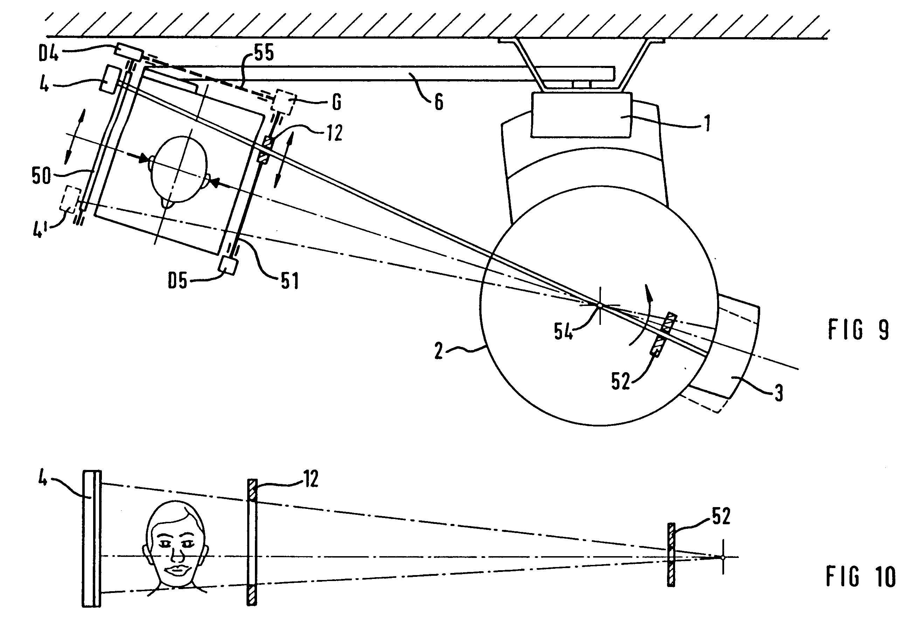

Bei der Ausführung gemäß Figur 9 wird die Primärblende nicht bewegt. Sie läuft vielmehr starr mit der Dreheinheit 2 um deren Drehmittelpunkt 54 um. Auch bei dieser Version ist es denkbar, die beiden Längsführungen 50 und 51 durch eine weitere Spindel und ein geeignetes, dazwischen gesetztes Untersetzungsgetriebe G miteinander zu synchronisieren.

Die Figur 10 zeigt eine mögliche Ausführung einer Strahlausrichtung, nämlich eine asymmetrische Einstellung des Strahles in bezug auf das zu durchstrahlende Objekt. Anstelle der asymmetrischen Einstellung ist auch eine symmetrische Ausrichtung des Fächerstrahls möglich.

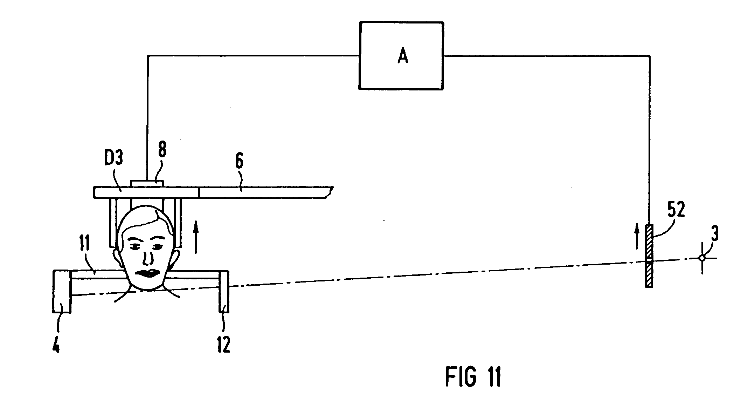

Die Figur 11 zeigt eine weitere vorteilhafte Ausführungsform, bei der die Kamera 4, wie in Figur 2 gezeigt, horizontal angeordnet und über einen Querträger 11 mit der Vorblende 12 verbunden ist. Kamera 4 und Vorblende 12 werden gemeinsam über einen Antrieb A mit der Primärblende 52 motorisch verstellt, wobei die Verstellung, wie beschrieben, im TDI-Modus erfolgt. Die Strahlenquelle 3 bleibt bei dieser Ausführungsform während der Verstellbewegung von Kamera und Primärblende ortsfest.

Wie aus einem Vergleich der Vorrichtung, wie sie in der eingangs zitierten EP-A-0 229 308 beschrieben ist, mit den Ausführungsformen der zuvor beschriebenen Figuren 1 bis 11 hervorgeht, ist gegenüber den herkömmlichen, mit Röntgenfilm und Verstärkungsfolien arbeitenden Geräten die Filmkassette durch einen elektronischen Strahlungswandler (2-dimensionaler Zeilendetektor) ersetzt. Der relativ schmale, dort eingesetzte Sensor empfängt einen vom Röntgenstrahler abgegebenen waagerechten Fächerstrahl, wobei die Anordnung von Strahler und Zeilendetektor so getroffen ist, daß sie durch beispielsweise eine Höhenverstellung des Gerätestativs vertikal am Objekt vorbeibewegt wird. Der Fächerstrahl wird dabei zunächst grob durch die strahlernahe Primärblende und danach durch eine unmitttelbar vor dem Objekt plazierte, in der vertikalen Bewegung mitbewegte Objekt- oder Vorblende scharf begrenzt.

Für die Tomosynthese muß das Objekt, im Ausführungsbeispiel der zu untersuchende Schädel, aus wenigstens zwei, vorteilhafterweise aus noch weiteren Winkelrichtungen durchstrahlt werden, wobei zur Erzielung einer optimalen Verwischung die Projektionen nicht in einer, sondern in verschiedenen Ebenen liegen sollten. Praktikabel scheint, wie dies nachfolgend anhand der verschiedenen Ausführungsformen dargestellt ist, die Projektionsrichtung nach oben, unten, links und rechts zu verändern.

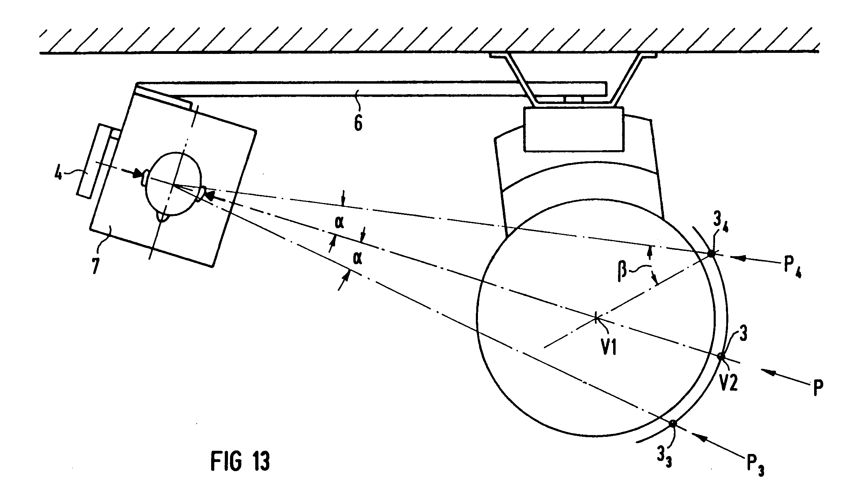

Die Figuren 12 und 13 zeigen in Anlehnung an die Ausführungsform gemäß Figur 2 eine erste Modifikation, bei der nicht nur, wie beschrieben, Strahlenquelle 3 und Zeilendetektorkamera 4 zusammen mit Vorblende 12 in der Höhe, also um das Maß h, in vertikaler Richtung verstellbar angeordnet sind, sondern bei der darüber hinaus, der hier nur symbolisch angedeutete Strahler 3, ausgehend von einer festen, waagerechten Projektionsrichtung P, seitlich und senkrecht verfahren wird. Figur 12 zeigt das senkrechte Verfahren des Strahlers. Der Strahler wird dabei auf einer vorgegebenen Kreisbahn mit dem Radius R, der dem Abstand zwischen Strahler-Fokus und Schädelmitte-Patient entspricht, um eine horizontale Achse geschwenkt. Das Verschwenken erfolgt um ein bestimmtes Winkelmaß α, der etwa zwischen 5 bis 15° liegen kann. Ein Objektpunkt ( OP )des zu durchstrahlenden Objekts (Schädel) wird also nicht nur aus der einen (üblichen) Projektionsrichtung P, sondern noch aus weiteren, in anderen Ebenen liegenden Projektionsrichtungen (P₁, P₂, P₃, P₄...) durchstrahlt , wobei der Strahlenfächer jeweils auf das Objekt mit der dahinterliegenden Zeilendetektorkamera 4 gerichtet ist. Die Objektblende 12 kann vorteilhafterweise entsprechend linear mit verschoben werden. Obgleich der Zeilendetektor in seiner Position verbleiben kann, kann es vorteilhaft sein, Objektblende und Strahlendetektor mitzudrehen. Dadurch erhält man eine saubere Ausblendung an den Blendenkanten und eine optimale Strahlendetektion.

In Betrachtung der Figur 13, die das seitliche Verfahren des (hier ebenfalls nur symbolisch angedeuteten) Strahlers 3 aufzeigt, ist erkennbar, daß der Strahler, wenn er von der Position P aus nach P₃ oder P₄ geschwenkt wird, auch noch um eine vertikale Achse V gedreht wird. Der Drehwinkel β ist so bemessen, daß in den Projektionsrichtungen P₃ und P₄ stets eine senkrechte Durchstrahlung des Objekts gegeben ist.

In Abwandlung der erläuterten Ausführungsform könnte anstelle der Abtastung mittels der Zeilendetektorkamera 4 in der zuvor beschriebenen Slot-Technik mit streifenweisem Aufbau des Flächenbildes auch eine großflächige Kamera mit einem Sensor auf der Basis von amorphem Silizium verwendet werden. Dadurch könnte das streifenweise Abtasten, also die gesamte Höhenverstellung von Strahler, Objektblende und Detektor, zur Erstellung eines 2D-Bildes entfallen.

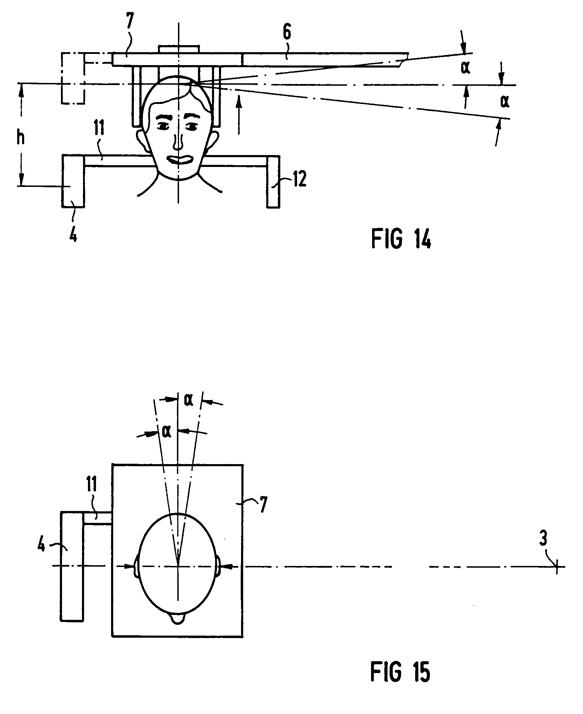

Die Figuren 14 und 15 zeigen weitere Ausführungsformen. Im Gegensatz zu dem zuvor beschriebenen Ausführungsbeispiel wird die Strahlenquelle 3 mit Ausnahme der Höhenverstellung zur Abtastung des Schädels nicht weiter verstellt. Strahler, Blenden und Zeilendetektor bleiben hier also zueinander fixiert, während das Objekt zur Erstellung der tomosynthetischen Aufnahmen gekippt wird. Hierzu kann vorteilhafterweise der gesamte Kopfhalter 7 gegenüber der Tragstange 6 um den Winkelbetrag α nach oben und unten gekippt (Fig. 14, bzw. nach links und rechts geschwenkt (Fig. 15) werden. Bei diesen Verstellvarianten kann man die ohnehin bereits vorhandenen Einstellmöglichkeiten für die Ohroliven und die Nasenstütze verwenden.

Eine vorteilhafte Variante der bisher erläuterten Ausführungsformen kann darin bestehen, die für CEPH-Aufnahmen vorgesehene Kopfhalte- und Positioniereinrichtung 7 an einem ortsfesten, geräteexternen Teil, z.B. an einer Raumwand, und die Zeilendetektor-Kamera 4 am höhenverstellbaren Auslegerarm 6 zu befestigen.

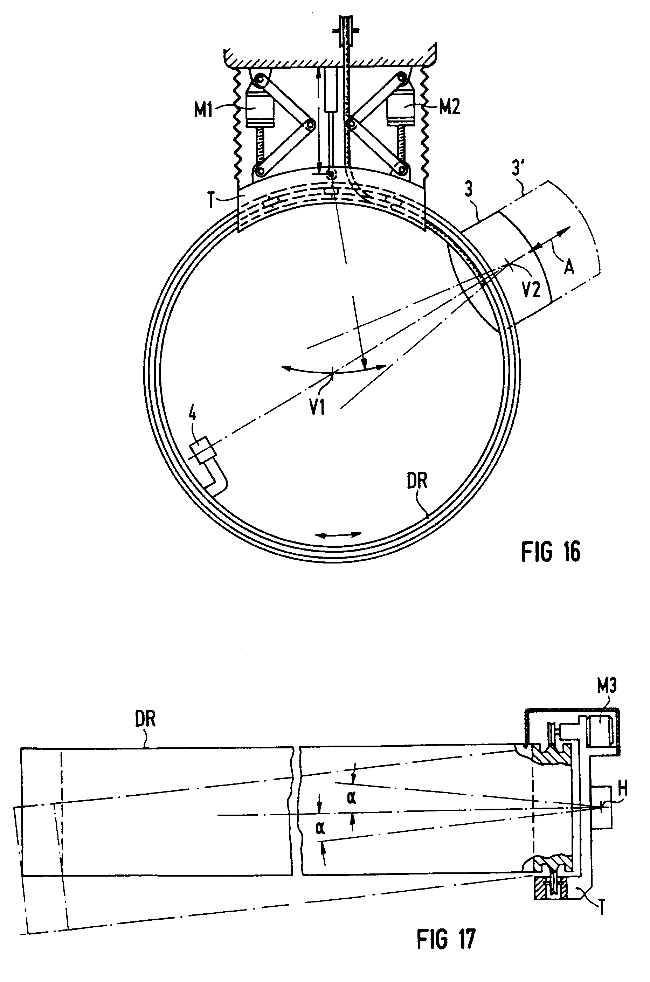

Die Modifikationen gemäß den Figuren 16 und 17 basieren auf der Vorrichtung, wie sie in der eingangs bereits genannten EP-0 229 308 beschrieben ist. Anstelle der Filmkassette ist eine Zeilendetektorkamera 4 vorgesehen, die, wie in Fig. 1 dargestellt , vertikal angeordnet ist. Die Aufnahmen für eine Tomosynthese in Slot-Technik erfolgt durch lineares, horizontales Verschieben der Anordnung Strahler 3 und Sensor 4 gegen das Objekt, indem der Sensor 4 und der Strahler 3 durch jeweilige Aktuatoren (Motoren M1, M2 oder M3) am Drehring (DR) gleichsinnig bewegt werden oder indem der Drehring komplett gegen das Objekt verschoben wird. Vorteilhafterweise wird der Strahler 3 um eine strahlerferne erste Vertikalachse (V1 in Figuren 13 und 16) auf einer vorgegebenen Kreisbahn geschwenkt und gleichzeitig und gegensinnig um eine strahlernahe zweite Vertikalachse (V2 in Figuren 13 und 16) gedreht. Vorteilhaft kann es auch sein, bei am Drehring fixiertem Strahler den Sensor zu schwenken, z.B. am Ring entlang um den Röhrenfokus als Drehpunkt. Des weiteren ist es denkbar, den Sensor linear zu verstellen, wobei entweder nur die (strahlernahe) Primärblende entsprechend mitbewegt oder Blende und Strahler mitgedreht werden.

Unterschiedliche tomosynthetische Projektionswinkel in der Horizontalen können durch Drehen des Ringes um das Objekt eingestellt werden, und zwar mit gleicher Mechanik, wie sie für Panorama-Aufnahmen erforderlich sind. Die verschiedenen Projektionswinkel in vertikaler Richtung können durch Taumeln des Ringes um eine Horizontalachse H (Fig. 17) erzielt werden, wobei bei dieser Taumel- oder Kippbewegung der Sensor zweckmäßigerweise mitbewegt wird.

Eine weitere Variante zu diesem Ausführungsbeispiel wäre, den Sensor für die Tomosynthese nicht vertikal, sondern horizontal anzuordnen. Für PAN- oder Ceph-Aufnahmen kann der Sensor entsprechend umpositioniert werden.

Unter den geometrischen Verhältnissen (Abstände, Winkel) der üblichen Anordnung für Panoramaaufnahmen würde der Kopf des Patienten in der Höhe nur teilweise erfaßt werden. Wenn, wie bei Ceph-Aufnahmen sinnvoll, der Schädel voll erfaßt werden soll, kann man dies z.B. durch nacheinander erstellte, höhenversetzte Bilder, die im Rechner nachträglich paßgenau zusammengesetzt werden, erreichen. Auch ist es denkbar, eine spezielle, unter größerem nutzbaren Winkel abstrahlende Röntgenröhre in Verbindung mit einem entsprechend längeren bzw. größeren Sensor zu verwenden. Besonders vorteilhaft ist es, den Strahler für diese Aufnahmen am Ring radial zu verstellen und so in einen größeren Abstand zum Objekt zu bringen. In der Fig. 16 ist diese Verstellmöglichkeit durch einen Doppelpfeil (A) am Strahler 3 angedeutet.

Bei den Lösungen gemäß den Figuren 12 bis 15, die dazu dienen, Ceph-Aufnahmen, also Aufnahmen vom gesamten Schädel eines Patienten, machen zu können, befinden sich Schädel und Röntgensensor üblicherweise entfernt vom Panoramagerät, das den Strahler beinhaltet. Der Grund hierfür ist, Verzerrung durch die Zentralprojektion klein zu halten. Verkürzt man den Ausleger, wachsen entsprechend die Verzerrungen, d.h. etwa bei einer Seitenaufnahme des Schädels wird die strahlernahe Kieferhälfte größer dargestellt als die strahlerferne.

Eine Extremsituation entsteht, wenn auf den Ausleger ganz verzichtet wird und der Röntgensensor an der Stelle, wie er bei der Panoramaaufnahme üblich, angeordnet wird (Figur 1).

Erfindungsgemäß lassen sich auch mit einer solchen Anordnung Schädelaufnahmen erstellen und tomosynthetisch aufbereiten, wenn man die Schichtbilder in Schichten strahlernah bis strahlerfern berechnet.

Da der Abstand des Zeilendetektors vom Strahler bekannt ist, können die Verzerrungen rechnerisch korrigiert werden. Die Bilder können als unverzerrte Schichtbilder oder 3D-Bilder wiedergegeben werden oder wieder zum gewohnten unverzerrten Summenabsorptionsbild rechnerisch zusammengesetzt und wiedergegeben werden.

Diese Lösung ist deshalb vorteilhaft, da hierbei kein gesonderter CEPH-Aufbau mit Ausleger gebraucht wird.

## Patentansprüche

1. Zahnärztliche Röntgendiagnostikeinrichtung zur Erstellung von tomosynthetischen Aufnahmen von Objekten, insbesondere von Schädel eines Patienten, bestehend aus einem Panorama-Aufnahmegerät mit einem Röntgenstrahler (3) und einer diametral gegenüber dem Strahler angeordneten Aufnahmeeinheit (4) in Form einer elektronischen Zeilendetektor-Kamera, **dadurch gekennzeichnet,** daß Mittel vorhanden sind, die es erlauben, den Röntgenstrahler (3) definiert in der Höhe zu verstellen und um eine senkrechte (V) und waagerechte Achse (H) derart zu verschwenken, daß ein Objektpunkt (OP) aus mehreren, vorzugsweise in unterschiedlichen Ebenen liegenden Projektionsrichtungen (P, P₁, P₂ ...) durchstrahlt wird.

2. Röntgendiagnostikeinrichtung nach Anspruch 1, **dadurch gekennzeichnet,** daß zum Einstellen der Projektionsrichtungen (P₁, P₂ ...) der Strahler (3) um eine Horizontalachse (H) verstellbar gehaltert ist (Fig. 12), und die Verstellbewegung des Strahlers (3) mit einer objektnahen Vorblende (12), gegebenenfalls auch mit der Aufnahmeeinheit (4) gekoppelt ist.

3. Röntgendiagnostikeinrichtung nach einem der Ansprüche 1 bis 2, **dadurch gekennzeichnet,** daß der Strahler (3) um eine strahlerferne erste Vertikalachse (V1) auf einer vorgegebenen Kreisbahn schwenkbar und gleichzeitig gegensinnig um eine strahlernahe Zweite Vertikalachse (V2) drehbar angeordnet ist (Fig. 13).

4. Röntgendiagnostikeinrichtung nach Anspruch 1, **dadurch gekennzeichnet,** daß zum Einstellen dar Projektionsrichtungen (P₁, P₂ ...) der Objekthalter (7) kippbar bzw. schwenkbar gehaltert ist (Fig. 14, 15).

5. Röntgendiagnostikeinrichtung nach Anspruch 1, **dadurch gekennzeichnet,** daß die diametral des Strahlers (3) angeordnete Aufnahmeeinheit (4) auf einem Drehring (DR) angeordnet ist, der einerseits um eine zentrale Vertikalachse (V3) drehbar und andererseits durch Verstellmittel (M1, M2) um eine weitere Vertikalachse (V4) schwenkbar gehaltert ist, und daß der Strahler (3) seinerseits um eine dritte Vertikalachse (V5) drehbar am Drehring (DR) gehaltert ist.

6. Röntgendiagnostikeinrichtung nach Anspruch 4, **dadurch gekennzeichnet,** daß der Drehring (DR) an einem Träger (T) gelagert ist, der die genannte Horizontalachse (H) zum Einstellen der Projektionsrichtungen aufnimmt (Fig. 17).

7. Röntgendiagnostikeinrichtung nach einem der Ansprüche 1 bis 6, **dadurch gekennzeichnet,** daß die Zeilendetektor-Kamera (4) einen hinter einer schlitzförmigen Öffnung (18) angeordneten Röntgenstrahlen-Detektor (35) beinhaltet, dessen Breite der Breite bzw. Länge des aufzunehmenden Körperteils angepaßt ist, wobei Verstellmittel (D1, D4) vorhanden sind, welche die Zeilendetektor-Kamera gegenüber dem Körperteil so verstellen, daß die Schlitzöffnung entlang des Körperteils bewegt wird, wobei der von der Strahlenblende (52, 12) der Strahlenquelle (3) begrenzte Fächerstrahl synchron zur Kamerabewegung mitbewegt wird.

8. Röntgendiagnostikeinrichtung nach Anspruch 7, bei der der Röntgenstrahlen-Detektor ein CCD-Sensor ist, der aus ein oder mehreren Zeilen aufgebaut ist und eine vorgesetzte Szintillationsschicht enthält.

9. Röntgendiagnostikeinrichtung nach einem der Ansprüche 1 bis 8, bei der die Zeilendetektor-Kamera (4) so gehaltert ist, daß die Schlitzöffnung (18) waagerecht verläuft und die Verstellbewegungg in senkrechter Richtung erfolgt (Fig. 2, 12 bis 15).

10. Röntgendiagnostikeinrichtung nach Anspruch 9, bei der die Zeilendetektor-Kamera (4) einem Auslegerarm (6) zugeordnet ist, der höhenverstellbar an einem die Strahlenquelle (3) tragenden Stativ (1) gehaltert ist (Fig. 2, 12, 13).

11. Röntgendiagnostikeinrichtung nach einem der Ansprüche 1 bis 8, bei der die Zeilendetektor-Kamera (4) so gehaltert ist, daß die Schlitzöffung (18) vertikal verläuft und die Verstellbewegung in waagerechter Richtung erfolgt (Fig. 1, 8, 9, 16, 17).

12. Röntgendiagnostikeinrichtung nach Anspruch 11, bei der die Strahlenquelle (3) verstellbar angeordnet ist und diese zusammen mit der Primärblende (52) um den Drehmittelpunkt (54) einer die Strahlenquelle tragenden Dreheinheit (2) verstellbar angeordnet ist (Fig. 9).

13. Röntgendiagnostikeinrichtung nach einem der Ansprüche 1 bis 12, bei der dem Panorama-Aufnahmegerät zur Erstellung von Kieferaufnahmen eines Patienten ein Ceph-Gerät zur Erstellung von Schädelfernaufnahmen eines Patienten zugeordnet ist, wobei das Panorama-Aufnahmegerät mit einer ersten, strahlernahen Kopfhalte- und Positioniereinrichtung (5) und einer ersten Halterung (15) für die Zeilendetektor-Kamera (4), und das Ceph-gerät mit einer zweiten, strahlerfernen Kopfhalte- und Positioniereinrichtung (7) und einer zweiten Halterung (15) für die Zeilendetektor-Kamera (4) versehen sind und beide Halterungen gleich ausgebildet sind.

14. Röntgendiagnostikeinrichtung nach Anspruch 13, welche zur Erstellung der Panorama-Aufnahmen und der Schädelernaufnahmen folgende Elemente beinhaltet:
- ein Stativ (1), mit einem höhenverstellbaren Tragteil (1a);
- eine Dreheinheit (2) mit der Röntgenstrahlenquelle (3) und der diametral dazu angeordneten ersten Kopfhalte- und Positioniereinrichtung (5),
- die erste Halterung (15) für die Zeilendetektor-Kamera (4) zur Erstellung der Panorama-Schichtaufnahmen
- einen Auslegerarm (6), an dem die zweite Kopfhalte- und Positioniereinrichtung (7) sowie die zweite Halterung (15) angeordnet sind, wobei beide Halterungen baugleiche Anschlußteile (24) für die alternative Halterung der vorgenannten Zeilendetektor-Kamera (4) aufweisen,
wobei die erste Halterung so angeordnet ist, daß der Zeilendetektor mit seiner Längsausdehnung vertikal ausgerichtet ist und gemeinsam mit der Strahlenquelle (3) um den Patientenkopf herum bewegbar ist, während die zweite Halterung so angeordnet ist, daß die Zeilendetektor-Kamera mit ihrer Längsausdehnung horizontal ausgerichtet ist und in bezug auf die Strahlenquelle in vertikaler Ebene verstellbar angeordnet ist.

15. Röntgendiagnostikeinrichtung nach Anspruch 14, bei der die zweite Kopfhalte- und Positioniereinrichtung (7) an einem ortsfesten, geräteexternen Teil befestigbar ist und die Zeilendetektor-Kamera (4) am höhenverstellbaren Auslegerarm (6) befestigt ist.

16. Röntgendiagnostikeinrichtung nach Anspruch 14, bei der die zweite Kopfhalte- und Positioniereinrichtung (7) am höhenverstellbaren Auslegerarm (6) angeordnet ist, und zwischen dem Auslegerarm und der zweiten Kopfhalte- und Positioniereinrichtung eine Einrichtung (10, 13, D3) vorgesehen ist, welche die Bewegung des höhenverstellbaren Tragteils (1a) während der Ceph-Aufnahme kompensiert.

17. Röntgendiagnostikeinrichtung nach Anspruch 1, dadurch **gekennzeichnet,** daß zur Erzeugung der Bilder von Fernröntgen-Aufnahmen die Aufnahmeeinheit (4) objektnah (Fig. 2, Fig. 15) angeordnet ist, daß ein Rechnervorhanden ist, in dem die tomosynthetischen Schichtbilder aus den vorgegebenen Abständen zwischen Strahler (3), Objekt und Aufnahmeeinheit (4) errechnet werden, und daß der Rechner ein Rechenprogramm enthält, mit dem eine durch die Zentralprojektion entstehende Abbildungsverzerrung elektronisch korrigiert wird.

## Claims

1. Dental X-ray diagnostic device for taking tomosynthetic images of objects, in particular of the skull of a patient, comprising a panorama radiographic device with an X-ray machine (3) and a radiographic unit (4), arranged diametrically opposite the source, in the form of an electronic line scanning detector camera, characterized in that means are present which permit the X-ray machine (3) to be adjusted in height in a defined way, and to be pivoted about a vertical axis (V) and a horizontal axis (H) in such a way that an object point (OP) is radiographed from a plurality of projection directions (P, P_{1,} P₂ ...) preferably lying in different planes.

2. X-ray diagnostic device according to Claim 1, characterized in that for the purpose of setting the projection directions (P₁, P₂ ...), the source (3) is held such that it can be adjusted about a horizontal axis (H) (Figure 12), and the adjusting movement of the source (3) is coupled to a preliminary diaphragm (12) near the object, and also, as appropriate, to the radiographic unit (4).

3. X-ray diagnostic device according to one of Claims 1 to 2, characterized in that the source (3) is arranged in a fashion capable of pivoting about a first vertical axis (V1), remote from the source, on a prescribed circular track, and in a fashion capable of simultaneously rotating in the opposite direction about a second vertical axis (V2), remote from the source (Figure 13).

4. X-ray diagnostic device according to Claim 1, characterized in that the object holder (7) is held in a fashion capable of tilting and/or pivoting in order to set the projection directions (P₁, P₂ ...) (Figures 14, 15).

5. X-ray diagnostic device according to Claim 1, characterized in that the radiographic unit (4) arranged diametrically relative to the source (3) is arranged on a rotating ring (DR) which is held in a fashion capable of rotating about a central vertical axis (V3), on the one hand, and of being pivoted about a further vertical axis (V4) by adjusting means (M1, M2), on the other hand, and in that, for its part, the source (3) is held on the rotating ring (DR) in a fashion capable of rotating about a third vertical axis (V5).

6. X-ray diagnostic device according to Claim 4, characterized in that the rotating ring (DR) is mounted on a support (T) which holds the said horizontal axis (H) for setting the projection directions (Figure 17).

7. X-ray diagnostic device according to one of Claims 1 to 6, characterized in that the line scanning detector camera (4) includes an X-ray detector (35) which is arranged behind a slit-shaped opening (18) and whose width is matched to the width or length of the body part to be imaged, there being present adjusting means (D1, D4) which adjust the line scanning detector camera with respect to the body part such that the slit opening is moved along the body part, the fan beam limited by the beam diaphragm (52, 12) of the radiation source (3) being co-moved synchronously with the camera movement.

8. X-ray diagnostic device according to Claim 7, in which the X-ray detector is a CCD sensor which is constructed from one or more rows and contains a scintillation layer situated in front.

9. X-ray diagnostic device according to one of Claims 1 to 8, in which the line scanning detector camera (4) is held such that the slit opening (18) runs horizontally, arid the adjusting movement is performed in a vertical direction (Figures 2, 12 to 15).

10. X-ray diagnostic device according to Claim 9, in which the line scanning detector camera (4) is assigned to a crossarm (6) which is held in a height-adjustable fashion on a stand (1) supporting the radiation source (3) (Figures 2, 12, 13).

11. X-ray diagnostic device according to one of Claims 1 to 8, in which the line scanning detector camera (4) is held such that the slit opening (18) runs vertically and the adjusting movement is performed in a horizontal direction (Figures 1, 8, 9, 16, 17).

12. X-ray diagnostic device according to Claim 11, in which the radiation source (3) is arranged adjustably, and the latter is arranged together with the primary diaphragm (52) such that it can be adjusted about the centre of rotation (54) of the rotary unit (2) supporting the radiation source (Figure 9).

13. X-ray diagnostic device according to one of Claims 1 to 12, in which for the purpose of taking images of a patient's jaw the panorama radiographic device is assigned a cephalographic device for taking remote skull images of a patient, it being the case chat the panorama radiographic device is provided with a first head-holding and positioning device (5) near the source and with a first holder (15) for the line scanning detector camera (4), and that the Ceph machine is provided with a second head-holding and positioning device (7), remote from the source, and with a second holder (15) for the line scanning detector camera (4), and that the two holders are of identical construction.

14. X-ray diagnostic device according to Claim 13, which contains the following elements for the purpose of taking the panorama images and the remote skull images:
- a stand (1) with a height-adjustable supporting part (1a);
- a rotary unit (2) with the radiation source (3) and the first head-holding and positioning device (5), arranged diametrically thereto,
- the first holder (15) for the line scanning detector camera (4) for the purpose of taking panorama tomographs, and
- a crossarm (6) on which the second head-holding and positioning device (7) and the second holder (15) are arranged, the two holders having connecting parts (24) of identical construction for the alternative holding of the abovenamed line scanning detector camera (4),
the first holder being arranged such that the line scanning detector is vertically aligned with its longitudinal extent and can be moved around the patient's head together with the radiation source (3), while the second holder is arranged such that the line scanning detector camera is horizontally aligned with its longitudinal extent and is arranged with reference to the radiation source such that it can be adjusted in a vertical plane.

15. X-ray diagnostic device according to Claim 14, in which the second head-holding and positioning device (7) can be fastened on a stationary part outside the device, and the line scanning detector camera (4) is fastened on the height-adjustable crossarm (6).

16. X-ray diagnostic device according to Claim 14, in which the second head-holding and positioning device (7) is arranged on the height-adjustable crossarm (6), and there is provided between the crossarm and the second head-holding and positioning device a device (10, 13, D3) which compensates the movement of the height-adjustable supporting part (1a) during the cephalographic imaging.

17. X-ray diagnostic device according to Claim 1, characterized in that for the purpose of producing the images of remote X-ray radiography, the radiographic unit (4) is arranged near the object (Figure 2, Figure 15), in that a computer is present in which the tomosynthetic tomographs are computed from the prescribed spacings between the source (3), object and radiographic unit (4), and in that the computer contains a computer program by means of which an image distortion produced by the central projection is corrected electronically.

## Revendications

1. Dispositif de diagnostic aux rayons X, utilisé dans le domaine dentaire pour des prises de vue de tomosynthèse d'objets, notamment du crâne d'un patient, le dispositif consistant en un appareil de prise de vues panoramiques avec une source de rayons X (3) et une unité de prise de vues (4), située de manière diamétralement opposée à la source de rayonnement et se présentant sous la forme d'une caméra électronique à balayage de ligne, caractérisé en ce que des moyens sont prévus pour permettre le déplacement en hauteur, d'une manière définie, de la source de rayons X (3) et son pivotement autour d'un axe vertical (V) et d'un axe horizontal (H) de telle manière qu'un point objet (OP) puisse être examiné à partir de plusieurs directions de projection (P, P₁, P₂ ...), situées de préférence dans des plans différents.

2. Dispositif de diagnostic aux rayons X selon la revendication 1, caractérisé en ce que, pour le réglage des directions de projection (P₁, P₂ ...), la source de rayonnement (3) est maintenue de manière mobile autour d'un axe horizontal (H) (figure 12), et en ce que le mouvement de déplacement de la source de rayonnement (3) est couplé à un avant-écran (12) proche de l'objet et, le cas échéant, également à l'unité de prise de vues (4).

3. Dispositif de diagnostic aux rayons X selon l'une des revendications 1 et 2, caractérisé en ce que la source de rayonnement (3) est placée de manière à pouvoir pivoter autour d'un premier axe vertical (V1) distant de la source, suivant une trajectoire circulaire prédéfinie, et de manière à pouvoir simultanément tourner en sens inverse autour d'un deuxième axe vertical (V2) proche de la source (figure 13).

4. Dispositif de diagnostic aux rayons X selon la revendication 1, caractérisé en ce que, pour le réglage des directions de projection (P₁, P₂ ...), l'élément de maintien d'objet (7) est maintenu de manière à pouvoir basculer ou bien à pouvoir pivoter (figures 14, 15).

5. Dispositif de diagnostic aux rayons X selon la revendication 1, caractérisé en ce que l'unité de prise de vues (4), située de manière diamétralement opposée à la source de rayonnement (3), est placée sur un anneau rotatif (DR) qui, d'une part, est maintenu de manière à pourvoir tourner autour d'un axe vertical central (V3) et qui, d'autre part, est maintenu, à l'aide de moyens de réglage (M1, M2), pour pouvoir pivoter autour d'un autre axe vertical (V4), et en ce que la source de rayonnement (3) est, quant à elle, maintenue contre l'anneau rotatif (DR) de manière à pouvoir tourner autour d'un troisième axe vertical (V5).

6. Dispositif de diagnostic aux rayons X selon la revendication 4, caractérisé en ce que l'anneau rotatif (DR) est monté sur un support (T) qui contient ledit axe horizontal (H) pour le réglage des directions de projection (figure 17).

7. Dispositif de diagnostic aux rayons X selon l'une des revendications 1 à 6, caractérisé en ce que la caméra à balayage de ligne (4) contient un détecteur de rayons X (35), qui est placé derrière une ouverture (18) en forme de fente, et dont la largeur est adaptée à la largeur ou bien à la longueur de la partie du corps à radiographier, des moyens de réglage (D1, D4) étant prévus pour déplacer la caméra à balayage de ligne par rapport à la partie du corps, de telle manière que l'ouverture en forme de fente soit déplacée le long de la partie du corps, tandis que le faisceau en éventail, limité par l'écran (52, 12) de la source de rayonnement (3), est déplacé en même temps de manière synchrone avec le mouvement de la caméra.

8. Dispositif de diagnostic aux rayons X selon la revendication 7, caractérisé en ce que le détecteur de rayons X est un capteur à CCD (à couplage de charges), qui est constitué à partir d'une ou de plusieurs ligne(s), et devant lequel est placée une couche à scintillation.

9. Dispositif de diagnostic aux rayons X selon l'une des revendications 1 à 8, dans lequel la caméra à balayage de ligne (4) est maintenue de telle manière que l'ouverture (18) en forme de fente s'étende horizontalement et que le mouvement de déplacement s'effectue dans le sens vertical (figures 2, 12 à 15).

10. Dispositif de diagnostic aux rayons X selon la revendication 9, dans lequel la caméra à balayage de ligne (4) est associée à une potence (6), qui est maintenue, de manière réglable en hauteur, sur un pied (1) portant la source de rayonnement (3) (figures 2, 12, 13).

11. Dispositif de diagnostic aux rayons X selon l'une des revendications 1 à 8, dans lequel la caméra à balayage de ligne (4) est maintenue de telle manière que l'ouverture (18) en forme de fente s'étende verticalement et que le mouvement de déplacement s'effectue dans le sens horizontal (figures 1, 8, 9, 16, 17).

12. Dispositif de diagnostic aux rayons X selon la revendication 11, dans lequel la source de rayonnement (3) est disposée de manière mobile, et dans lequel celle-ci est disposée de manière mobile, en même temps que l'écran primaire (52), autour du centre de rotation (54) d'une unité rotative (2) portant la source de rayonnement (figure 9).

13. Dispositif de diagnostic aux rayons X selon l'une des revendications 1 à 12, dans lequel un appareil de prise de vues du crâne, servant à la téléradiographie du crâne d'un patient, est associé à l'appareil de prise de vues panoramiques, servant à obtenir des vues panoramiques des mâchoires d'un patient, et dans lequel l'appareil de prise de vues panoramiques est pourvu d'un premier dispositif de positionnement et de maintien de tête (5), proche de la source de rayonnement, et d'une première console (15) pour la caméra à balayage de ligne (4), tandis que l'appareil de prise de vues du crâne est pourvu d'un deuxième dispositif de positionnement et de maintien de tête (7), distant de la source de rayonnement, et d'une deuxième console (15) pour la caméra à balayage de ligne (4), les deux consoles étant de structure identique.

14. Dispositif de diagnostic aux rayons X selon la revendication 13, qui, pour l'obtention des vues panoramiques et des téléradiographies du crâne, contient les éléments suivants :
- un pied (1), comportant une partie porteuse (1a) réglable en hauteur ;
- une unité rotative (2) avec la source de rayons X (3) et le premier dispositif de positionnement et de maintien de tête (5), diamétralement opposé ;
- la première console (15) pour la caméra à balayage de ligne (4) pour l'obtention de tomographies panoramiques ;
- une potence (6) sur laquelle sont placés le deuxième dispositif de positionnement et de maintien de tête (7) ainsi que la deuxième console (15), les deux consoles présentant des éléments de raccordement (24) de conception identique pour le support alternatif de ladite caméra à balayage de ligne (4) ;
la première console étant disposée de telle manière que la caméra à balayage de ligne soit dirigée verticalement par son étendue longitudinale et qu'elle puisse être déplacée, en même temps que la source de rayonnement (3) autour de la tête d'un patient, tandis que la deuxième console est disposée de telle manière que la caméra à balayage de ligne soit dirigée horizontalement par son étendue longitudinale et qu'elle soit mobile dans un plan vertical par rapport à la source de rayonnement (3).

15. Dispositif de diagnostic aux rayons X selon la revendication 14, dans lequel le deuxième dispositif de positionnement et de maintien de tête (7) peut être fixé sur une partie fixe, externe à l'appareil, et dans lequel la caméra à balayage de ligne (4) est fixée sur la potence (6) réglable en hauteur.

16. Dispositif de diagnostic aux rayons X selon la revendication 14, dans lequel le deuxième dispositif de positionnement et de maintien de tête (7) est disposé contre la potence (6) réglable en hauteur, tandis qu'on prévoit, entre la potence et le deuxième dispositif de positionnement et de maintien de tête, un dispositif (10, 13, D3) qui compense le mouvement de la partie porteuse (1a) réglable en hauteur, pendant la prise de vues du crâne.

17. Dispositif de diagnostic aux rayons X selon la revendication 1, caractérisé en ce que, pour l'obtention des images de téléradiographie, l'unité de prise de vues (4) est disposée proche de l'objet (figure 2, figure 15), en ce qu'un calculateur est présent, dans lequel sont calculées les tomographies de tomosynthèse à partir des distances prédéfinies entre source de rayonnement (3), objet et unité de prise de vues (4), et en ce que le calculateur contient un programme de calcul, au moyen duquel est corrigée électroniquement une distorsion d'image provoquée par la projection centrale.
